# EUROPEAN PATENT SPECIFICATION

(11) **EP 2 574 364 B1**
(45) Date of publication and mention of the grant of the patent: **02.12.2020**
(21) Application number: 12184079.7
(22) Date of filing: 12.09.2012
(51) Int. Cl.: A61M 25/00, A61M 1/36

(54) **Catheter with tapering surfaces**
Katheter mit spitz zulaufenden Oberflächen
Cathéter avec surfaces coniques

(30) Priority: 29.09.2011 US 201113248548
(43) Date of publication of application: 03.04.2013
(73) Proprietor: Covidien LP, Mansfield, MA 02048 (US)
(72) Inventor: Deshpande, Manish, Canton, MA Massachusetts 02021 (US)
(74) Representative: Gray, James

(56) References cited:
- WO-A1-2009/052506
- US-A- 2 972 779
- US-A1- 2009 118 661
- US-A1- 2009 192 435

## Description

### BACKGROUND

### 1. Technical Field

The present disclosure relates generally to medical catheters, and more particularly to catheters having tapering surfaces.

### 2. Description of the Related Art

Catheters are flexible medical instruments for use in the introduction and withdrawal of fluids to and from body cavities, ducts and vessels. Catheters are used for many different applications within the human body including the administration of liquid therapeutic agents and the removal of bodily fluids for testing, monitoring, or disposal. Catheters have a particular application in hemodialysis procedures, in which blood is withdrawn from a blood vessel, directed to a hemodialysis unit for dialysis or purification, and subsequently returned to the blood vessel.

Typically, dialysis catheters define at least two lumens including a venous lumen and an arterial lumen. The arterial lumen withdraws blood from the patient and delivers the blood to a dialyzer. The venous lumen receives purified blood from the dialyzer and returns the blood to the patient. The venous and arterial lumens may include distal openings adjacent the tip of the catheter. In addition, the venous and arterial lumens may also include side openings which provide redundant or alternate flow paths to and from the arterial and venous lumens.

The efficiency of a hemodialysis procedure may be reduced by recirculation of blood flow at a distal end of the catheter. Recirculation occurs when dialyzed blood exiting the venous lumen is drawn directly back into the arterial lumen of the catheter. To overcome this drawback, some catheter devices stagger the openings of the catheter lumens such that the opening of the venous lumen is disposed distally beyond the opening of the arterial lumen. These catheter devices, however, also suffer from various additional drawbacks. For example, the staggered openings of the venous lumen and arterial lumen render the catheter less suitable for reversing fluid flow through the catheter. Reversibility of fluid flow through the catheter may be used to remove the formation of thrombus within the opening of the catheter. Thus, the staggered openings may disadvantageously indirectly result in a higher likelihood of flow occlusion within the catheter.

Therefore, it would be desirable to overcome the disadvantages and drawbacks of the prior art with a multiple lumen catheter that minimizes the likelihood of recirculation without negatively affecting the ability to reverse flow in the catheter. It would also be highly desirable if the catheter and its constituent parts are easily and efficiently manufactured and assembled.

US 2009/192435A1, WO 2009/052506A1, US 2009/118661A1, US 2972779A describe catheters.

In particular US 2009/192435A1 discloses, in e.g. figure 28A, a catheter body 11 which comprises a distal tip region 1220, which includes a venous lateral opening 1242 and an arterial lateral opening 1244, each defined by the catheter body. A venous distal opening 1332A and an arterial distal opening 1332B are also included in the distal tip region 1220, as defined by the catheter body 11. The venous distal opening 1332A is defined as to be relatively larger than the venous distal opening 1232A of FIG. 26A, while the arterial distal opening 1332B is also defined to be larger relative to the arterial distal opening 1232B shown in FIG. 26A. The relatively larger sizes of the distal openings provide for enhanced fluid flow there through. As best seen in FIG. 28B, the distal profile of the distal tip region 1220 is relatively less tapered when compared to the profile of FIG. 25A, though it is appreciated that the profile and relative sizes of the lateral and distal openings of the distal tip region can be modified in various ways, as appreciated by one skilled in the art.

### SUMMARY OF THE DISCLOSURE

Accordingly, the present disclosure is directed to a catheter having an elongated tubular body and a septum. The elongated tubular body defines a longitudinal axis and includes a first wall defining a first lumen and a second wall defining a second lumen. The first lumen and the second lumen communicate with first and second distal openings, respectively. The septum separates the first and second lumens, and extends beyond the first and second distal openings. One or both of the first and second walls includes a side opening.

The side opening is in fluid communication with one of the first and second lumens. The side opening has an external aperture and an internal aperture. The internal aperture is smaller in dimension than the external aperture. The side opening is substantially frustoconical in shape.

Each side opening may be defined by one or more sidewalls. Each sidewall may taper inwardly from the external aperture to the internal aperture such that the dimension of the side opening adjacent the external aperture is greater than the dimension of the side opening adjacent the internal aperture.

The first wall may define a first side opening and the second wall may define a second side opening. The first side opening may be in fluid communication with the first lumen. The second side opening may be in fluid communication with the second lumen. The first and second side openings may be longitudinally aligned along the longitudinal axis of the elongated tubular body. The first and second side openings may be longitudinally offset along the longitudinal axis of the elongated tubular body.

The first wall may define a first internal surface and a first external surface. The second wall may define a second internal surface and a second external surface. The first internal surface may define the first lumen and the second internal surface may define the second lumen.

### SUMMARY OF THE INVENTION

According to the present invention there is provided a catheter comprising: an elongated tubular body defining a longitudinal axis, the elongated tubular body including a first wall defining a first lumen and a second wall defining a second lumen, the first lumen and the second lumen communicating with first and second distal openings, respectively; and a septum separating the first and second lumens, and extending beyond the first and second distal openings; at least one of the first and second walls defining a side opening in fluid communication with one of the first and second lumens, the side opening having an external aperture and an internal aperture smaller in dimension than the external aperture and wherein the side opening is substantially frustoconical in shape.

### BRIEF DESCRIPTION OF THE DRAWINGS

The objects and features of the present disclosure, which are believed to be novel, are set forth with particularity in the appended claims. The present disclosure, both as to its organization and manner of operation, together with further objectives and advantages, may be best understood by reference to the following description, taken in connection with the accompanying drawings, as set forth below.
FIG. 1 is a perspective view of one embodiment of a presently disclosed catheter in accordance with the principles of the present disclosure;
FIG. 2 is a cross-sectional view of the presently disclosed catheter shown in FIG. 1;
FIG. 3 is a perspective view of an alternate example of the presently disclosed catheter in accordance with the principles of the present disclosure;
FIG. 4A is a cross-sectional view of the presently disclosed catheter shown in FIG. 3;
FIGS. 4B-4C are enlarged cross-sectional views of the indicated areas of detail delineated in FIG. 4A;
FIGS. 5A-5C are cross-sectional views of the presently disclosed catheter shown in FIG. 4A taken along the indicated areas of detail delineated in FIG. 4A; and
FIG. 6A is a cross-sectional view of another example of the presently disclosed catheter in accordance with the principles of the present disclosure; and
FIGS. 6B-6C are enlarged cross-sectional views of the indicated areas of detail delineated in FIG. 6A.

### DETAILED DESCRIPTION OF THE EXEMPLARY EMBODIMENTS

The exemplary embodiments of the catheter and methods of use (not claimed) lisclosed are discussed in terms of medical catheters for the administration of fluids into and out of the body of a subject and more particularly, in terms of a catheter including a catheter tip that limits undesirable recirculation during use to facilitate unobstructed fluid flow. The catheter is advantageously configured to facilitate reversible fluid flow between lumens thereof. The present disclosure may be employed with a range of catheters, such as, for example, hemodialysis, peritoneal, infusion, PICC, CVC, and port and catheter applications including surgical, diagnostic and related treatments of diseases and body ailments of a subject.

In the discussion that follows, the term "proximal" will refer to the portion of a structure that is closer to a practitioner, while the term "distal" will refer to the portion that is further from the practitioner. According to the present disclosure, the term "practitioner" refers to a doctor, nurse or other care provider and may include support personnel. As used herein, the term "subject" refers to a human patient or other animal.

The following discussion includes a description of the catheter, in accordance with the principles of the present disclosure. Reference will now be made in detail to the exemplary embodiments of the disclosure, which are illustrated in the accompanying figures.

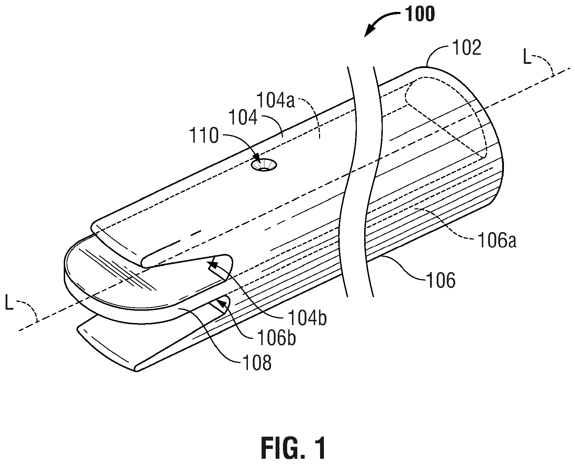

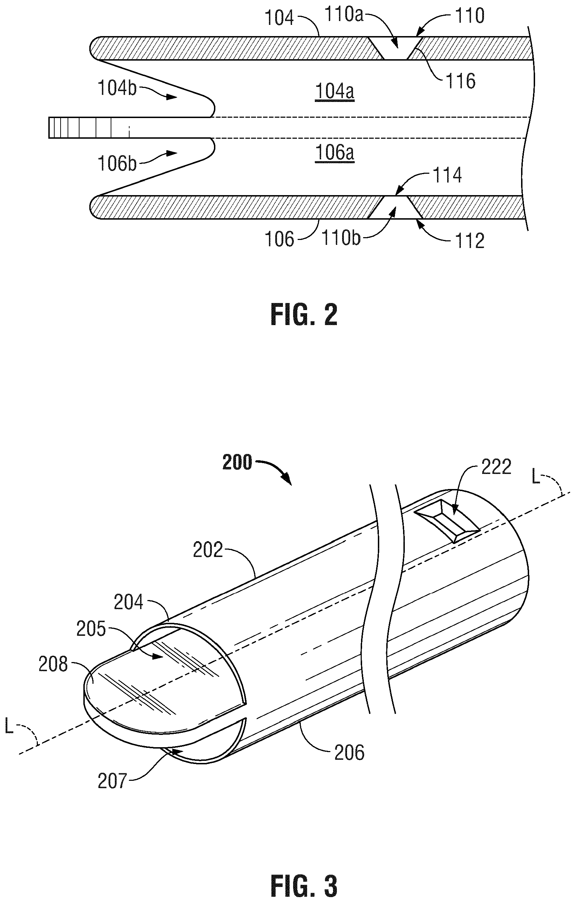

Referring to FIGS. 1-2, one embodiment of the presently disclosed catheter is shown which is generally referred to as catheter 100. Catheter 100 includes an elongated tubular body 102 which defines a longitudinal axis "L" and includes a first wall 104, a second wall 106, and a septum 108. A first lumen 104a is defined between first wall 104 and septum 108. The first lumen 104a extends to a first distal opening 104b. A second lumen 106a is defined between second wall 106 and septum 108. The second lumen 106a extends to a second distal opening 106b. Septum 108 separates first and second lumens 104a, 106a and extends distally beyond first and second distal openings 104b, 106b. One or both of first and second walls 104, 106 includes a side opening 110. In one embodiment, as shown in FIG. 2, a first side opening 110a is defined in first wall 104 and is disposed in fluid communication with first lumen 104a. A second side opening 110b is defined in second wall 106 and is disposed in fluid communication with second lumen 106a. First and second side openings 110a, 110b are disposed proximally of distal openings 104b and 106b and may be longitudinally aligned or longitudinally offset along the longitudinal axis "L" of the elongated tubular body 102 and may have any suitable dimension and/or shape (e.g., sinusoidal, circular, polygonal, etc.).

Each side opening 110 has an external aperture 112 (FIG. 2) and an internal aperture 114. Internal aperture 114 is smaller in dimension than the external aperture 112. In this regard, internal and external apertures 114, 112 are interconnected by a sidewall or sidewalls 116 which define each side opening 110. As shown in FIGS. 1 and 2, sidewall 116 is angled or tapers inwardly from external aperture 112 to internal aperture 114 such that the dimension of the side openings 110a and 110b is greatest adjacent an external surface of the wall 104, 106 of catheter 100 and smallest adjacent an internal surface of the wall 104, 106 of the catheter 100. Thus, first and second side openings 110a, 110b provide increased flow resistance to fluid flowing from within lumen 104a or 106a to a location externally of catheter 100 and decreased flow resistance to fluid flowing into a lumen 104a or 106a of catheter 100. Because the side openings 110a and 110b are configured to provide increased flow resistance to fluid exiting the catheter 100, fluid exiting first lumen 104a or second lumen 106a will tend to exit lumen 104a or 106a through a distal opening 104b or 106b, respectively. Conversely, because side openings 110a and 110b are configured to provide decreased flow resistance to fluid entering catheter 100, fluid entering catheter 100 will tend to enter catheter 100 through a side opening 110a or 110b and not through distal opening 104b or 106b. As a result, the spacing between the primary fluid flow stream exiting the catheter 100 through the distal opening 104b or 106b and the primary fluid flow stream entering the catheter 100 through side openings 110a and 110b is increased to minimize the likelihood of fluid recirculation between the arterial and venous lumens of the catheter 100.

In one embodiment, sidewalls 116 may be substantially frustoconical as depicted in FIGS. 1 and 2. As will be discussed in greater detail, the side openings, namely sidewalls 116 defining the side openings 110, may be disposed at various orientations and may have any suitable dimension and/or shape. In some embodiments, various internal surfaces of the side openings may be disposed at a plurality of different angles relative to internal and external surfaces of the elongated tubular body.

With reference now to FIGS. 3-4, another example of the presently disclosed catheter is shown which is generally referred to as catheter 200. Catheter 200 is substantially similar to catheter 100 but is described herein only to the extent necessary to describe the differences in construction and operation thereof. Catheter 200 includes an elongated tubular body 202 which defines a longitudinal axis "L" and includes a first wall 204, a second wall 206, and a septum 208.

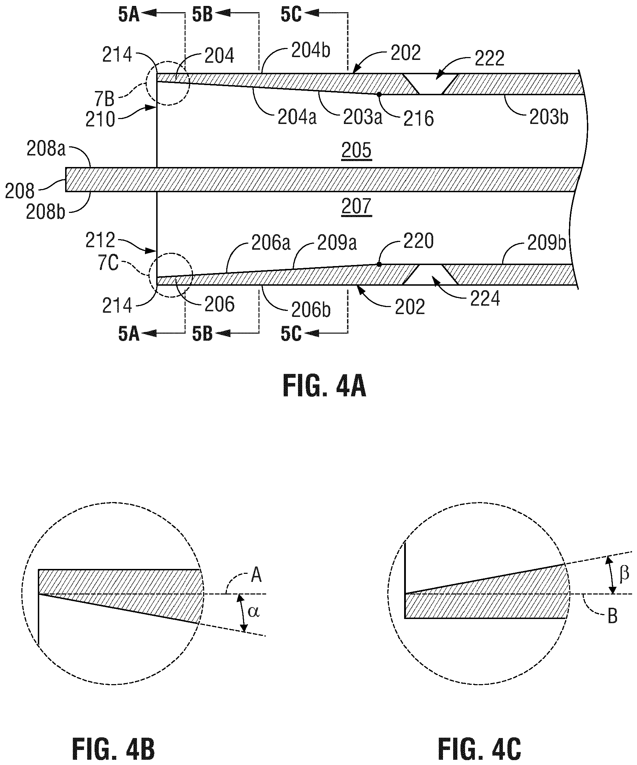

As best depicted in FIG. 4A, first wall 204 defines a first side opening 222 and includes a first internal surface 204a and a first external surface 204b. Second wall 206 defines a second side opening 224 and includes a second internal surface 206a and a second external surface 206b. First and second side openings 222, 224 may be substantially linear or have any other suitable shape (e.g., sinusoidal, circular, polygonal, etc.) as discussed above with respect to side openings 110a and 110b. First internal surface 204a of first wall 204 and septum 208 define first lumen 205. Lumen 205 includes a first distal flow portion 203a and a first proximal flow portion 203b. The second internal surface 206a of second wall 206 and septum 208 define second lumen 207. Lumen 207 includes a second distal flow portion 209a and a second proximal flow portion 209b. First internal surface 204a tapers proximally from a first distal opening 210 defined in a distal end of elongated tubular body 202 along first distal flow portion 203a to a point 216. Point 216 defines the proximal end of first distal flow portion 203a and the distal end of first proximal flow portion 203b. More particularly, first distal flow portion 203a is the region disposed between first distal opening 210 and point 216 and first proximal flow portion 203b is the region disposed proximal of point 216.

First distal opening 210 is defined in the elongated tubular body 202 between first internal surface 204a of first wall 204 and a top surface 208a of septum 208. Second internal surface 206a tapers proximally from a second distal opening 212 defined in the distal end of elongated tubular body 202 to a point 220. Point 220 defines the proximal end of second distal flow portion 209a and the distal end of second proximal flow portion 209b. More particularly, second distal flow portion 209a is the region disposed between second distal opening 212 and point 220 and second proximal flow portion 209b is the region disposed proximal of point 220. Second distal opening 212 is defined in elongated tubular body 202 between second internal surface 206a of second wall 206 and a bottom surface 208b of septum 208.

To this end, first internal surface 204a tapers proximally at an angle α relative to longitudinal axis "L" (e.g., relative to a line "A" which is parallel to longitudinal axis "L"; FIG. 4B) of elongated tubular body 202 to point 216 along first distal flow portion 203a. As such, the dimension of lumen 205 increases in the distal direction in the first distal flow portion 203a. First internal surface 204a may be substantially parallel to longitudinal axis "L" proximal of point 216 along first proximal flow portion 203b.

Similarly, second internal surface 206a tapers proximally at an angle β relative to longitudinal axis "L" (e.g., relative to a line "B" which is parallel to longitudinal axis "L"; FIG. 4C) of elongated tubular body 202 to point 220 along second distal flow portion 209a. As such, the dimension of lumen 207 increases in a distal direction in the second distal flow portion 209a. Second internal surface 206a is substantially parallel to longitudinal axis "L" proximal of point 220 along second proximal flow portion 209b. Points 216 and 220 may be longitudinally aligned and/or longitudinally offset.

Each of first and second distal flow portions 203a, 209a are configured to provide increased flow resistance to fluid flowing into catheter 200 through distal openings 210 and 212 and decreased flow resistance to fluid flowing out from catheter 200 through distal openings 210 and 212. As illustrated, side opening 222 which communicates with lumen 205 and side opening 224 which communicates with lumen 207 are each configured, as discussed above with respect to side openings 110a and 110b, to have dimensions which decrease from the external surface of catheter 200 towards the internal surface of catheter 200. As such, side openings 222 and 224 are configured to provide increased flow resistance to fluid flowing from catheter 200 through a side opening 222 or 224 of catheter 200 and to provide decreased flow resistance to fluid flowing through side opening 222 and 224 into catheter 200.

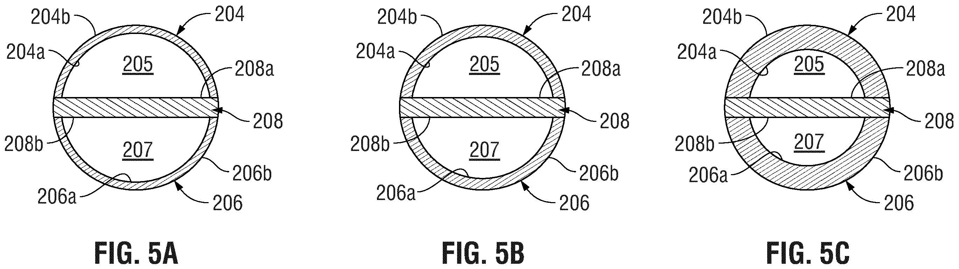

As shown in FIGS. 5A-5C, the cross-sectional dimension of each lumen 205 and 207 increases in the distal direction from points 216 and 220 to the distal end of catheter 200. Although first internal surface 204a and second internal surface 206a are illustrated as being substantially linear, surfaces 204a and 206a may have non-linear or curved configurations in the longitudinal direction or any other configuration which increases the dimension of lumens 205 and/or 207 in the distal direction in the first and second distal flow portions 203a and 209a.

Due to the combined configurations of the first distal flow portion 203a and the second distal flow portion 209a, and the configuration of the side openings 222 and 224, fluid tends to flow into the catheter 200 through a side opening 222 or 224 of an arterial lumen and out of the catheter through a distal opening 210 or 212 of the venous lumen. Because of this, the spacing of the primary fluid stream exiting catheter 200 and the primary fluid stream entering the catheter 200 is maximized to minimize the likelihood of recirculation of fluid from the arterial lumen to the venous lumen of catheter 200.

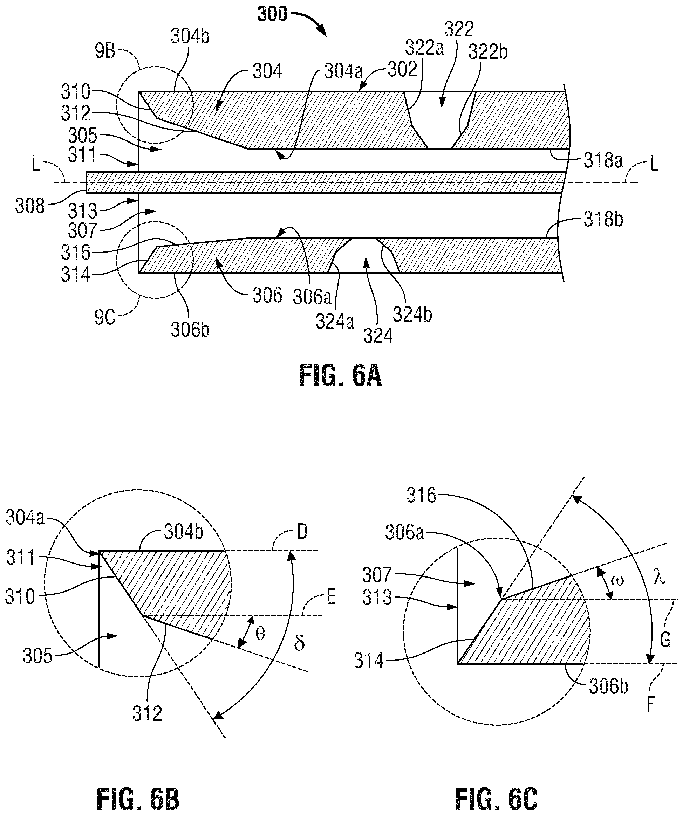

With reference now to FIG. 6A, another example of the presently disclosed catheter is shown which is generally referred to as catheter 300. Catheter 300 is substantially similar to catheters 100 and 200 but is described herein only to the extent necessary to describe the differences in construction and operation thereof. Catheter 300 includes an elongated tubular body 302 which defines a longitudinal axis "L" and includes a first wall 304, a second wall 306, and a septum 308.

As best depicted in FIGS. 6A-6C, first wall 304 defines a first side opening 322 and includes a first internal surface 304a and a first external surface 304b. A first lumen 305 is defined between first wall 304 and septum 308. Second wall 306 defines a second side opening 324 and includes a second internal surface 306a and a second external surface 306b. A second lumen 307 is defined between second wall 306 and septum 308.

First internal surface 304a of first wall 304 defines a first distal tapering surface 310 and a first proximal tapering surface 312. First distal tapering surface 310 of first internal surface 304a tapers proximally from a first distal opening 311 of first lumen 305 to first proximal tapering surface 312 of first internal surface 304a at a first leading angle δ relative to a line "D" (FIG. 6B) that is parallel to the longitudinal axis "L" of elongated tubular body 302. First proximal tapering surface 312 of first internal surface 304a tapers proximally from the proximal end of first distal tapering surface 310 of first internal surface 304a to a first proximal internal surface 318a of first internal surface 304a at a first trailing angle θ relative to a line "E" (FIG. 6B) that is parallel to longitudinal axis "L." As such, the dimension of lumen 305 increases in the distal direction in first distal tapering surface 310 and first proximal tapering surface 312 of first internal surface 304a. First leading angle δ and first trailing angle θ may be different or the same. First proximal internal surface 318a is substantially parallel to longitudinal axis "L" of elongated tubular body 302.

Second internal surface 306a of second wall 306 includes a second distal tapering surface 314 and a second proximal tapering surface 316. Second distal tapering surface 314 of second internal surface 306a tapers proximally from a second distal opening 313 of second lumen 307 to second proximal tapering surface 316 of second internal surface 306a at a second leading angle λ relative to a line "F" (FIG. 6C) that is parallel to longitudinal axis "L" of elongated tubular body 302. Second proximal tapering surface 316 of second internal surface 306a tapers proximally from the proximal end of second distal tapering surface 314 of second internal surface 306a to a second proximal internal surface 318b of second internal surface 306a at a second trailing angle ω relative to a line "G" (FIG. 6C) that is parallel to longitudinal axis "L." As such, the dimension of lumen 307 increases in the distal direction in second distal tapering surface 314 and second proximal tapering surface 316 of second internal surface 306a. Second leading angle λ and second trailing angle ω may be different or the same. Second proximal internal surface 318b of second internal surface 306a is substantially parallel to longitudinal axis "L" of elongated tubular body 302.

Although first internal surface 304a of first wall 304 and second internal surface 306a of second wall 306 are illustrated as being substantially linear, first and second internal surfaces 304a and 306a may have parabolic configurations in the longitudinal direction or any other configuration which increases the dimension of lumens 305 and/or 307 in the distal direction in first distal tapering surface 310, first proximal tapering surface 312, second distal tapering surface 314, and second proximal tapering surface 316, respectively.

As can be appreciated from FIG. 6A, first and second walls 304, 306 may each have a first thickness and a second thickness. The first and second thicknesses may be different. First and second side openings 322, 324 may be longitudinally offset and or aligned along the longitudinal axis "L" of the elongated tubular body 302 and may be any suitable shape and/or dimension and have any suitable angular orientation as discussed above with respect to side openings 110a, 110b, 222, and 224. In particular, as best illustrated in FIG. 6A, for example, first side opening 322 may include first and second interior surfaces 322a, 322b which may be disposed at different angles relative to one another. Similarly, second side opening 324 may include first and second interior surfaces 324a, 324b which may be disposed at different angles relative to one another.

Thus, catheter 300, by virtue of first and second internal surfaces 304a, 306a and side openings 322, 324 provide increased flow resistance to fluid flowing from within lumen 305 or 307 into a patient and decreased flow resistance to fluid flowing from a patient into a lumen 305 or 307 as discussed above.

Any of the presently disclosed surfaces and/or components of the presently disclosed catheters may be planar or non-planar, such as, for example, arcuate, undulating, textured, etc.

The components of the presently disclosed catheters are fabricated from materials suitable for medical applications, such as, for example, polymerics or metals, such as stainless steel, depending on the particular catheter application and/or preference of a practitioner. Semi-rigid and rigid polymerics are contemplated for fabrication, as well as resilient materials, such as molded medical grade polypropylene. One skilled in the art will realize that other materials and fabrication methods (fabrication methods not claimed) suitable for assembly and manufacture, in accordance with the present disclosure, also would be appropriate.

## Claims

1. A catheter (100), comprising:
an elongated tubular body (102) defining a longitudinal axis (L), the elongated tubular body (102) including a first wall (104) defining a first lumen (104a) and a second wall (106) defining a second lumen (106a), the first lumen (104a) and the second lumen (106a) communicating with first and second distal openings (104b, 106b), respectively; and
a septum (108) separating the first and second lumens (104a, 106a), and extending beyond the first and second distal openings (104b, 106b);
at least one of the first and second walls (104, 106) defining a side opening (110a, 110b) in fluid communication with one of the first and second lumens (104a, 106a), the side opening (110a, 110b) having an external aperture (112) and an internal aperture (114) smaller in dimension than the external aperture (112) and wherein the side opening (110) is substantially frustoconical in shape.

2. The catheter (100) of claim 1, wherein each side opening (110) is defined by at least one sidewall (116).

3. The catheter (100) of claim 1 or claim 2, wherein the sidewall (116) tapers inwardly from the external aperture (112) to the internal aperture (114) such that the dimension of the side opening (110a, 110b) adjacent the external aperture (112) is greater than the dimension of the side opening (110a, 110b) adjacent the internal aperture (114).

4. The catheter (100) of any of the preceding claims, wherein the first wall (104) defines a first side opening (110a) and the second wall (106) defines a second side opening (110b), the first side opening (110a) being in fluid communication with the first lumen (104a) and the second side opening (110b) being in fluid communication with the second lumen (106a).

5. The catheter (100) of claim 4, wherein the first and second side openings (110a, 110b) are longitudinally aligned along the longitudinal axis (L) of the elongated tubular body (102).

6. The catheter (100) of claim 4, wherein the first and second side openings (110a, 110b) are longitudinally offset along the longitudinal axis (L) of the elongated tubular body (102).

7. The catheter (100) of any of the preceding claims, wherein the first wall (104) defines a first internal surface and a first external surface, and wherein the second wall (106) defines a second internal surface and a second external surface, the first internal surface defining the first lumen (104a) and the second internal surface defining the second lumen (106a).

## Patentansprüche

1. Katheter (100), der Folgendes umfasst:
einen länglichen röhrenförmigen Körper (102), der eine Längsachse (L) definiert, wobei der längliche röhrenförmige Körper (102) eine erste Wand (104), die ein erstes Lumen (104a) definiert, und eine zweite Wand (106) beinhaltet, die ein zweites Lumen (106a) definiert, wobei das erste Lumen (104a) und das zweite Lumen (106a) mit einer ersten beziehungsweise einer zweiten distalen Öffnung (104b, 106b) in Verbindung stehen; und
ein Septum (108), das das erste und das zweite Lumen (104a, 106a) trennt und sich über die erste und die zweite distale Öffnung (104b, 106b) hinaus erstreckt;
die erste und/oder die zweite Wand (104, 106), die eine Seitenöffnung (110a, 110b) definieren, die mit einem des ersten und des zweiten Lumens (104a, 106a) in Fluidverbindung steht, wobei die Seitenöffnung (110a, 110b) einen Außendurchlass (112) und einen Innendurchlass (114) aufweist, deren Abmessung kleiner als der Außendurchlass (112) ist, und wobei die Seitenöffnung (110) im Wesentlichen eine kegelstumpfförmige Form aufweist.

2. Katheter (100) nach Anspruch 1, wobei jede Seitenöffnung (110) durch wenigstens eine Seitenwand (116) definiert ist.

3. Katheter (100) nach Anspruch 1 oder Anspruch 2, wobei sich die Seitenwand (116) von dem Außendurchlass (112) zu dem Innendurchlass (114) nach innen derart verjüngt, dass die Abmessung der Seitenöffnung (110a, 110b), die an den Außendurchlass (112) angrenzt, größer als die Abmessung der Seitenöffnung (110a, 110b) ist, die an den Innendurchlass (114) angrenzt.

4. Katheter (100) nach einem der vorhergehenden Ansprüche, wobei die erste Wand (104) eine erste Seitenöffnung (110a) definiert und die zweite Wand (106) eine zweite Seitenöffnung (110b) definiert, wobei die erste Seitenöffnung (110a) in Fluidverbindung mit dem ersten Lumen (104a) steht und die zweite Seitenöffnung (110b) in Fluidverbindung mit dem zweiten Lumen (106a) steht.

5. Katheter (100) nach Anspruch 4, wobei die erste und die zweite Seitenöffnung (110a, 110b) entlang der Längsachse (L) des länglichen röhrenförmigen Körpers (102) in Längsrichtung ausgerichtet sind.

6. Katheter (100) nach Anspruch 4, wobei die erste und die zweite Seitenöffnung (110a, 110b) entlang der Längsachse (L) des länglichen röhrenförmigen Körpers (102) in Längsrichtung versetzt sind.

7. Katheter (100) nach einem der vorhergehenden Ansprüche, wobei die erste Wand (104) eine erste Innenoberfläche und eine erste Außenoberfläche definiert, und wobei die zweite Wand (106) eine zweite Innenoberfläche und eine zweite Außenoberfläche definiert, wobei die erste Innenoberfläche das erste Lumen (104a) definiert und die zweite Innenoberfläche das zweite Lumen (106a) definiert.

## Revendications

1. Cathéter (100) comprenant :
un corps tubulaire allongé (102) définissant un axe longitudinal (L), le corps tubulaire allongé (102) comportant une première paroi (104) définissant une première lumière (104a) et une seconde paroi (106) définissant une seconde lumière (106a), la première lumière (104a) et la seconde lumière (106a) communiquant avec des première et seconde ouvertures distales (104b, 106b), respectivement ; et
une cloison (108) séparant les première et seconde lumières (104a, 106a) et s'étendant au-delà des première et seconde ouvertures distales (104b, 106b) ;
au moins une des première et seconde parois (104, 106) définissant une ouverture latérale (110a, 110b) en communication fluidique avec l'une des première et seconde lumières (104a, 106a), l'ouverture latérale (110a, 110b) ayant une ouverture externe (112) et une ouverture interne (114) de plus petite dimension que l'ouverture externe (112) et l'ouverture latérale (110) étant de forme sensiblement tronconique.

2. Cathéter (100) selon la revendication 1, dans lequel chaque ouverture latérale (110) est définie par au moins une paroi latérale (116).

3. Cathéter (100) selon la revendication 1 ou la revendication 2, dans lequel la paroi latérale (116) se rétrécit vers l'intérieur depuis l'ouverture externe (112) vers l'ouverture interne (114) de telle sorte que la dimension de l'ouverture latérale (110a, 110b) adjacente à l'ouverture externe (112) est supérieure à la dimension de l'ouverture latérale (110a, 110b) adjacente à l'ouverture interne (114).

4. Cathéter (100) selon l'une quelconque des revendications précédentes, dans lequel la première paroi (104) définit une première ouverture latérale (110a) et la seconde paroi (106) définit une seconde ouverture latérale (110b), la première ouverture latérale (110a) étant en communication fluidique avec la première lumière (104a) et la seconde ouverture latérale (110b) étant en communication fluidique avec la seconde lumière (106a).

5. Cathéter (100) selon la revendication 4, dans lequel les première et seconde ouvertures latérales (110a, 110b) sont alignées longitudinalement le long de l'axe longitudinal (L) du corps tubulaire allongé (102).

6. Cathéter (100) selon la revendication 4, dans lequel les première et seconde ouvertures latérales (110a, 110b) sont décalées longitudinalement le long de l'axe longitudinal (L) du corps tubulaire allongé (102).

7. Cathéter (100) selon l'une quelconque des revendications précédentes, dans lequel la première paroi (104) définit une première surface interne et une première surface externe, et dans lequel la seconde paroi (106) définit une seconde surface interne et une seconde surface externe, la première surface interne définissant la première lumière (104a) et la seconde surface interne définissant la seconde lumière (106a).
